# EUROPEAN PATENT APPLICATION

(11) **EP 1 136 467 A1**
(43) Date of publication of application: **26.09.2001**
(21) Application number: 00106398.1
(22) Date of filing: 24.03.2000
(51) Int. Cl.: C07C 11/02, C07C 5/46

(54) **Catalytic conversion of alkanes to alkenes**

(71) Applicant: AVENTIS ANIMAL NUTRITION S.A., 92164 Antony Cedex (FR)
(72) Inventor: Le Gendre, Olivier, 95220 Herblay (FR)
(74) Representative: Hoey, Shona

(57) **Abstract**

A process for the catalytic reaction of a C₂ to C₅ alkane and a sulphur containing compound to produce the corresponding alkene and hydrogen sulphide wherein the reaction mixture is contacted with a catalyst at a temperature of from 300 to 650°C wherein the catalyst has a surface area greater than 100 square metres per gram.

## Description

The present invention relates to a catalytic process for the conversion of an alkane to the corresponding alkene and in particular to a process for the cataytic conversion of an alkane and sulphur to the corresponding alkene and hydrogen sulphide.

The catalytic dehydrogenation of alkanes to alkenes is a well known reaction but the commercial operation of this reaction has suffered problems. In particular under the operating conditions used in this reaction there is often the unwanted side reaction of cracking of high alkane which results in the production ethene and methane. Furthermore, low conversion of the alkane leads to the necessary additional separation step to isolate the desired alkene product.

US Patent No 3801661 discloses a process for the dehydrogenation of non-aromatic C₃ to C₅ alkanes to the corresponding alkene. The hydrocarbon is contacted with a metal sulphide catalyst. The reaction requires the presence of hydrogen sulphide and steam. These components are not co-reactants but are essential to maintain the stability of the catalyst under the fairly severe operating conditions, in particular the reaction temperature which is 700°C. The conversion rate in this process is 70% and thus an additional separation step is necessary to isolate the alkane product.

US Patent No 3456026 discloses the sulphur dehydrogenation of organic compounds. In particular this patent discloses a process for the dehydrogenation of alkanes to alkenes. The process of this patent may be carried out at temperature in excess of 800°F although in reality the operating temperature is in excess of 1000°F. The catalyst used in the dehydrogenation has a surface are of between to 0.01 and 100 square metres per gram of catalyst. The patent states that the catalyst must have allow surface area to avoid cracking and tar formation.

We have found that high conversion of the alkane and high selectivity to the corresponding alkene can be achieved when the alkane is reacted with a sulphur containing compound in the presence of a catalyst under les severe reaction conditions and in particular with the specific combination of low temperature and a catalyst having a high surface area.

Accordingly, the present invention provides a process for the catalytic reaction of a C₂ to C₅ alkane and a sulphur containing compound to produce the corresponding alkene and hydrogen sulphide wherein the reaction mixture is contacted with a catalyst at a temperature of from 300 to 650°C wherein the catalyst has a surface area greater than 100 square metres per gram.

The process of the present invention provides the advantage over the prior art in that a high proportion of alkanes are converted to the corresponding alkene. Thus, there is no need for the additional separation step to isolate the alkene from the product stream. Furthermore, operation of the process at a relatively low temperature reduces the amount of unwanted side reactions.

The process of the present invention is directed to the reaction between a C₂ to C₅ alkane and a sulphur containing compound to produce the corresponding alkene and hydrogen sulphide. By the "corresponding alkene" is meant the unsaturated product having the same number of carbon atoms as the feed hydrocarbon. A particularly preferred alkane feed for use in the present invention is propane and thus the reaction between propane and a sulphur containing compound to provide propene and hydrogen sulphide is particularly preferred.

The process of the present invention may be carried out in the gas phase or in the liquid phase. It is preferred to carry out the process in the gas phase.

The sulphur containing compound used in the process of the present invention is a compound which is able to react with the alkane to yield hydrogen sulphide. Suitable sulphur containing compounds include sulphur oxides, namely sulphur dioxide and sulphur trioxide, H₂SO₃, H₂SO₄, ammonium sulphite, ammonium sulphate, elemental sulphur or a mixture thereof. The sulphur containing compound may be present in the reaction mixture in the liquid or gaseous form. Preferably, the sulphur containing compound is present as gaseous sulphur.

The molar ratio of sulphur to alkane is suitably from 0.1 to 10 moles to of sulphur to 1 mole of alkane, preferably from 0.2 to 5 moles of sulphur to 1 mole of alkane.

Inert diluents such as nitrogen, noble gases e.g. helium and argon, carbon dioxide, carbon monoxide, hydrogen sulphide, steam, methanol and carbon disulphide or a mixture thereof may be included in the reaction mixture. The inert diluent may be present in a total concentration of from 0 to 95%, preferably from 0 to 75% of the mixture.

A preferred reaction mixture is 10% propane, 15% gaseous sulphur and 75% nitrogen.

The catalyst used in the process of the present invention may be selected from the known dehydrogenation catalysts. Suitable dehydrogenation catalysts include metallic sulphides, in particular where the metal is a metal from Groups V B, VI B, VII B, VIII B and Group I B of the Periodic Table. Examples of sulphide catalysts include tungsten sulphide, nickel sulphide, molybdenum sulphide or cobalt sulphide. The metal sulphide catalyst may comprise a mixture of two or more metals. Suitable catalysts falling into this category include tungsten/nickel, molybdenum/nickel and molybdenum/cobalt sulphides. The preferred metal sulphide catalyst is a cobalt/molybdenum sulphide catalyst. The metal sulphide catalyst may be introduced into the reactor in the sulphide form or alternatively, may be introduced in another form which is capable of being converted to the sulphide form, for example the oxide form may be used and treated with a mixture of hydrogen and hydrogen sulphide prior to use.

Metal oxide catalysts may also be used in the process of the present invention and in particular oxides of Group VI B. Preferably, the metal oxide catalyst is chromium oxide. The metal oxide catalyst may comprise two or more metals and in particular a mixture of molybdenum and chromium is preferred.

The metal sulphide or metal oxide catalyst may be supported on a support. Suitable supports include alumina, titania, zirconia, silica, aluminosilicates or a mixture thereof. Preferably the catalyst support is alumina.

A further class of materials capable of catalysing the process of the present invention is carbon- based materials such as activated carbon. These materials optionally may be promoted with a suitable active material such as metal sulphides.

The catalyst used in the process of the present invention has a surface area greater than 100 square metres per gram. The actual surface area may vary according to the support or carrier used with the catalyst. For example where the catalyst is a metal sulphide or metal oxide, preferably, the catalyst has a surface area of greater than 100 and less than 400 m²/g, especially between 200 and 300 m²/g. Where the catalyst is carbon-based, the surface area may be greater that 100 m²/g and less than 600 m²/g

After a long periods of time in use, the catalyst may need to be regenerated. The regeneration may be carried out by passing gaseous sulphur over the catalyst at the reaction temperature for a suitable period of time. Typically, the sulphur is contacted with the catalyst for 10 to 15 hours.

A particular advantage of the present invention is that the process can be operated under mild reaction conditions. The process is operated at a temperature of from 300 to 650°C, more preferably from 400 to 500°C. Good conversion rates and yield of product are obtained when the process is operated at 450°C.

The process may be operated at any suitable pressure, for example below atmospheric, above atmospheric or at atmospheric pressure. Suitably, the process may be operated at a pressure of from 0.05 to 50 bar, preferably from 0.1 to 20 bar.

The weight space velocity is suitably from 50 to 5000 h⁻¹, preferably from 100 to 1000 h⁻¹. It will of course be apparent to the person skilled in the art that the space velocity will vary according to the temperature and pressure.

The process may be operated in any suitable reactor capable of handling the heat transfer to the catalyst bed. Suitable reactors include multi-tubular reactor, a standard reactor equipped with an internal heating coil or a simple adiabatic reactor.

The process may be operated batchwise, semi-continuously or continuously. It is preferred to operate the process as a continuous process.

The products of the present process are predominantly the alkane and hydrogen sulphide. Conversion of the alkane is typically 90 to 95%. Selectivity to the alkene is typically greater than 90%, preferably greater than 95%. A small amount of by-products are present in the product stream such as hydrogen, carbon disulphide, 1- and 2-propyl thiol mercaptan and dimers. These by-products are present in small quantities, typically from 100 to 50000 ppm volume and may be separated by distillation.

The present invention will now be illustrated with reference to the following example:

### Example 1: Conversion of Propane and Sulphur to Propene and Hydrogen Sulphide

A molybdenum/cobalt oxide catalyst commercially available from Procatalyse under the name TG103, having a surface area of 250m²/g, was treated with a mixture of hydrogen and hydrogen sulphide at a temperature not exceeding 350°C for 6 hours to provide the molybdenum/cobalt sulphide form.

A glass reactor was loaded with 300g of the catalyst. A gaseous mixture of sulphur and propane, molar ratio of 1.5 to 1, was fed into the reactor at a weight space velocity of 124.11h⁻¹. The gas flow rate was 37.23 kg per hour.

The reactor was heated to 450°C and the process operated under a pressure of 1.15 bar for approximately 10 hours on stream until a steady state temperature was reached.

The gaseous product stream was analysed by gas chromatography. The analyses gave 98.39% conversion of propane and 70.84% conversion of sulphur. The selectivity from the converted propane is given in the following Table 1.

**Table 1:**

| Selectivity from Converted Propane | |
|---|---|
| | SELECTIVITY (%) |
| Propene | 94.94 |
| CS₂ | 1.02 |
| Light cracking compounds (C₂H₄, CH₄) | 1.02 |
| Heavy compounds | 0.84 |
| CH₃SH | ot Detected |

The selectivity from the converted sulphur is given in the following Table 2

**Table 2:**

| Selectivity from Converted Sulphur | |
|---|---|
| | SELECTIVITY (%) |
| CH₃SH | Not Detected |
| H₂S | 94.26 |
| CS₂ | 5.65 |
| Other Sulphur Compounds | 0.1 |

This example shows that the an alkane and sulphur can be catalytically converted to the corresponding alkene and hydrogen sulphide under mild reaction conditions using a catalyst with a high surface area.

## Claims

**1.** A process for the catalytic reaction of a C₂ to C₅ alkane and a sulphur containing compound to produce the corresponding alkene and hydrogen sulphide wherein the reaction mixture is contacted with a catalyst at a temperature of from 300 to 650°C wherein the catalyst has a surface area greater than 100 square metres per gram.

**2.** A process as claimed in claim 1 carried out at a temperature of from 400 to 500°C.

**3.** A process as claimed in claim 1 or claim 2 in which the catalyst has a surface area of from 100 to 600 square metres per gram.

**3.** A process as claimed in any one of the preceding claims in which the alkane and the sulphur containing compound is present in a mole ratio of from 0.1 to 10 moles of sulphur to 1 mole of alkane.

**4.** A process as claimed in any one of the preceding claims in which the alkane is propane.

**5.** A process as claimed in any one of the preceding claims in which the sulphur containing compound is elemental sulphur.

**6.** A process as claimed in any one of the preceding claims in which the catalyst is a metallic sulphide wherein the metal is selected from Groups V B, VI B, VII B, VIII B and Group I B of the Periodic Table

**7.** A process as claimed in claim6 in which the catalyst is a metallic sulphide is selected from tungsten sulphide, nickel sulphide, molybdenum sulphide cobalt sulphide, tungsten/nickel sulphide, molybdenum/nickel sulphide and molybdenum/cobalt sulphide or a mixture thereof.

**8.** A process as claimed in claim 7 in which the catalyst is molybdenum/cobalt sulphide.

**9.** A process as claimed in any one of claims 1 to 5 in which the catalyst is a metal oxide wherein the metal is selected from Group VI B of the Periodic Table.

**10.** A process as claimed in claim 9 in which the catalyst is chromium oxide.

**11.** A process as claimed in any one of the preceding claims in which the catalyst comprises a support selected from alumina, titania, zirconia, silica, aluminosilicates or a mixture thereof.

**12.** A process as claimed in any one of claims 1 to 5 in which the catalyst is a carbon-based catalyst.

**13.** A process as claimed in claim 12 in which the catalyst is promoted with an active material.

**14.** A process as claimed in any one of the preceding claims carried out under a pressure of from 0.05 to 50 bar and a weight hourly space of from 50 to 5000 h⁻¹.
